(19) Europäisches Patentamt
European Patent Office
Office européen des brevets

(11) Numéro de publication : **0 310 527 B1**

# (12) FASCICULE DE BREVET EUROPEEN

(45) Date de publication du fascicule du brevet : **12.02.92 Bulletin 92/07**

(51) Int. Cl.$^5$ : **C07C 209/60,** C07C 209/14, C07C 211/03

(21) Numéro de dépôt : **88420323.3**

(22) Date de dépôt : **27.09.88**

## (54) Procédé de fabrication d'amines à partir d'oléfines.

(30) Priorité : **02.10.87 FR 8713873**

(43) Date de publication de la demande : **05.04.89 Bulletin 89/14**

(45) Mention de la délivrance du brevet : **12.02.92 Bulletin 92/07**

(84) Etats contractants désignés : **AT BE CH DE ES FR GB GR IT LI LU NL SE**

(56) Documents cités :
**EP-A- 0 049 463**
**EP-A- 0 200 923**
**GB-A- 502 737**

(73) Titulaire : **ATOCHEM**
**4 & 8, Cours Michelet La Défense 10**
**F-92800 Puteaux (FR)**

(72) Inventeur : **Brigandat, Yves**
**7, rue Fiorello Micolini**
**F-69120 Vaulx-en-Velin (FR)**
Inventeur : **Kervennal, Jacques**
**134, rue E. Locard**
**F-69005 Lyon (FR)**

**EP 0 310 527 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un procédé de fabrication d'amines par réaction à température élevée d'une monooléfine avec l'ammoniac ou une amine primaire ou secondaire en présence d'un catalyseur.

Il est connu de réaliser une telle réaction selon par exemple les procédés décrits dans les demandes de brevet européen EP-0039061 et EP-0200923. Le premier consiste à opérer à une température comprise entre 100°C et 250°C en présence d'un catalyseur à base de ruthenium ou de fer dissous en milieu solvant. Pratiquement il ne paraît convenir qu'à la mise en oeuvre de l'éthylène et présente l'inconvénient de ne pas procurer de façon sélective une amine déterminée. Le second consiste à opérer à une température comprise de préférence entre 250°C et 350°C en présence d'un halogénure d'ammonium comme catalyseur. Ce dernier est préférablement accompagné par un promoteur à base de métal de transition sans activité catalytique propre et qui peut être soit un halogénure de métal de transition soit un sel d'ammonium d'un oxyacide de métal de transition.

Selon les brevets des Etats Unis d'Amérique US-4459191 et US-4483757, un halogénure d'ammonium convient encore pour obtenir les amines à partir d'oléfines par photocatalyse en phase liquide.

Ainsi, parmi les composés comportant un groupe ammonium $NH_4$, l'art antérieur distingue-t-il spécifiquement les halogénures d'ammonium.

Il a maintenant été trouvé que des composés à groupement $NH_4$ autres que les halogénures conviennent particulièrement bien pour obtenir une amine de façon effective et sélective à partir d'une monooléfine, de même qu'à partir de l'alcool correspondant à l'hydratation de l'oléfine ou de mélanges d'une oléfine et dudit alcool.

La présente invention a en effet pour objet un procédé de fabrication d'une amine par réaction à température élevée d'une monooléfine ou de l'alcool correspondant à son hydratation ou d'un mélange d'une monooléfine et dudit alcool, avec l'ammoniac ou une amine primaire ou secondaire, en présence d'un catalyseur et d'un solvant, caractérisé en ce que le catalyseur est le sulfate d'ammonium $(NH_4)_2SO_4$ ou un sulfate double d'ammonium et d'un métal de transition.

Le sulfate d'ammonium peut être mis en oeuvre en l'état ou par exemple se former à partir de l'ammoniac et d'acide sulfurique ou de sulfate acide d'ammonium.

Des sulfates doubles d'ammonium et d'un métal de transition qui conviennent pour l'invention sont par exemple ceux du type alun de formule générale $XNH_4(SO_4)_2$, $12H_2O$ dans laquelle X représente le métal de transition.

Le plus généralement le métal de transition est choisi parmi le chrome, le rhodium, le vanadium et le fer, ce dernier étant le plus souvent préféré aux autres.

Le catalyseur est mis en oeuvre en quantité généralement telle que le rapport molaire catalyseur/oléfine est compris entre environ 0,01 et 0,5.

Le rapport molaire $NH_3$ (ou amine primaire ou secondaire) / oléfine est le plus souvent compris entre 1/1 et 10/1, et de préférence au moins égal à 2/1.

La température à laquelle est réalisé le procédé de l'invention est généralement comprise entre environ 200°C et 450°C afin d'assurer une conversion de l'oléfine et une sélectivité en amine produite suffisamment élevées.

Pour une oléfine comportant 4 atomes de carbone dans sa molécule, comme par exemple l'isobutène, la température ne sera pas supérieure en général à environ 300°C.

La pression est la pression autogène fournie par le milieu de réaction dans les conditions choisies pour l'exécution du procédé. Dans le cas de l'isobutène par exemple elle n'est pratiquement pas inférieure à environ 100 bars absolus.

L'oléfine et l'amine qui, outre $NH_3$, est appelée à réagir avec elle sont choisies parmi celles communément citées à propos d'un procédé du type de celui de l'invention, par exemple dans la demande de brevet européen EP-0200923. Parmi les monooléfines auxquelles s'appliquent ainsi l'invention et qui ont de 2 à 8 atomes de carbone dans leur molécule, celles qui ont de 2 à 4 atomes de carbone dans leur molécule, tel l'isobutène, reste préférée.

Ce qui est énoncé ci-dessus pour l'oléfine vaut pour l'alcool correspondant à son hydratation comme pour l'oléfine et ledit alcool en mélange, par exemple pour l'isobutène, le tertiobutanol ou leurs mélanges.

L'ammoniac est souvent choisi de préférence à une amine pour des raisons de coût et de disponibilité, de sélectivité et d'importance des amines primaires.

Les solvants mis en oeuvre peuvent être du type de ceux cités dans la demande de brevet européen EP-0200923 mais l'eau, l'alcool correspondant à l'hydratation de l'oléfine, ou leurs mélanges, sont préférés.

L'oléfine, l'alcool, l'ammoniac ou l'amine récupérables au terme de l'opération selon l'invention sont évidemment recyclables avec profit.

La durée de l'opération selon l'invention dépend du choix de l'oléfine et des paramètres de réaction. Elle est souvent comprise entre environ quelques dizaines de minutes et une quinzaine d'heures, généralement entre 1 et 10 heures.

Les exemples suivants, donnés à titre indicatif mais non limitatif, illustrent l'invention.

Ils sont relatifs l'isobutène. L'amine formée est la tertiobutylamine

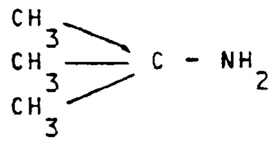

dont l'intérêt va croissant dans l'industrie en particulier dans le domaine phytosanitaire.

La conversion de l'oléfine en amine est exprimée en nombre de moles d'oléfine transformée en amine pour cent moles d'oléfine engagée.

La sélectivité en amine est exprimée par le rapport du nombre de moles d'oléfine transformée en amine au nombre total de moles d'oléfine transformée, pour cent moles d'oléfine engagée.

Lorsque l'alcool intervient il est confondu avec l'oléfine pour la détermination de la conversion et de la sélectivité, l'alcool et l'oléfine étant recyclables.

Il a été vérifié qu'en l'absence de catalyseur, il n'y a pas formation d'amine.

Exemple 1 :

Un mélange formé à partir de 90 g (5,3 moles) d'ammoniac anhydre, 56 g (1 mole) d'isobutène, 20 g (0,15 mole) de sulfate d'ammonium $(NH_4)_2SO_4$, et 360 g (20 moles) d'eau est maintenu à 250°C durant 10 heures sous agitation dans un autoclave de 1 l en acier inoxydable. La pression est la pression autogène.

Il est ainsi obtenu 25,6 g (0.35 mole) de tertiobutylamine, corrrespondant à une conversion de l'isobutène en amine de 35 %, à coté de l'isobutène non transformé et de 14.8 g (0.2 mole) de tertiobutanol.

Exemple 2 :

Dans l'autoclave de l'exemple 1, un mélange renfermant les mêmes quantités d'isobutène, d'ammoniac et d'eau que dans l'exemple 1, 6.6 g (0.05 mole) de sulfate d'ammonium, est maintenu à 240°C durant 8 heures sous agitation et sous pression autogène.

Il est alors obtenu 11 g (0,15 mole) de tertiobutylamine, correspondant à une conversion de l'isobutène en amine de 15 %, à côté de l'isobutène non transformé et de 16,3 g (0.22 mole) de tertiobutanol.

Exemple 3 :

L'exemple 2 est répéré à la différence près que la quantité de sulfate d'ammonium engagé est égale à 26,4 g (0,2 mole), que le mélange est maintenu à 240°C et ce seulement pendant 5 heures.

Il est ainsi obtenu 16.8 g (0.23 mole) de tertiobutylamine, correspondant à une conversion de l'isobutène de 23 %, à côté de l'isobutène non transformé et de 14.8 g (0.2 mole) de tertiobutanol.

Exemple 4 :

Un mélange formé à partir de 74 g (1 mole) de tertiobutanol, 90 g (5,3 moles) d'ammoniac anhydre, 40 g (0,3 mole) de sulfate d'ammonium, et 360 g (20 moles) d'eau, est maintenu sous agitation à 240°C durant 10 heures sous pression autogène dans l'autoclave de l'exemple 1.

Il est obtenu alors 22 g (0,3 mole) de tertiobutylamine, soit une conversion du tertiobutanol en amine de 30 %, et 32 g (0,57 mole) d'isobutène.

Exemple 5 :

L'exemple 1 est répété en engageant 28 g (0,5 mole) d'isobutène et 37 g (0,5 mole) de tertiobutanol.

Il est obtenu 33,6 g (0,46 mole) de tertiobutylamine soit une conversion cumulée de l'oléfine et du tertiobutanol de 46 %, à côté de 21,3 g (0,38 mole) d'isobutène tandis que subsistent 11,8 g (0,16 mole) de tertiobutanol.

A titre de comparaison, la répétition de l'exemple 5 en engageant $NH_4Cl$ à la place de $(NH_4)_2SO_4$, en quantité molaire égale, n'aboutit à la formation que de 20 g (0,27 mole) de tertiobutylamine.

Exemple 6 :

Un mélange formé à partir de 74 g (1 mole) de tertiobutanol, 90 g (5,3 moles) d'ammoniac anhydre, 9 g (0,07 mole de sulfate d'ammonium) sont maintenus sous agitation durant 5 heures à 240°C dans l'autoclave de l'exemple 1.

Il se forme 7,3 g (0,1 mole) de tertiobutylamine et 44,4 g d'isobutène.

Exemple 7 :

L'exemple 1 est répété aux différences suivantes près : Le sulfate double d'ammonium et de fer $NH_4Fe(SO_4)_2$ est mis en oeuvre à raison de 11,3 g (0,05 mole) à la place du simple sulfate d'ammonium et la durée du maintien en température à 250°C est de 5 heures au lieu de 10 heures.

Il est ainsi obtenu 14,5 g (0,2 mole) de tertiobutylamine, correspondant à une conversion de l'oléfine en amine de 20 %, à côté de l'isobutène non transformé et de 15 g (0,2 mole) de tertiobutanol.

Dans tous les exemples ci-dessus selon l'invention il n'a pas été constaté la formation de produits huileux et la sélectivité de la réaction en tertiobutylamine a toujours été pratiquement égale à 100 % ou très voisine de 100 % aux erreurs d'appréciation près.

## Revendications

1. Procédé de fabrication d'amines par réaction à

température élevée d'une monooléfine ou de l'alcool correspondant à son hydratation ou d'un mélange d'une monooléfine et dudit alcool, avec l'ammoniac ou une amine primaire ou secondaire, en présence d'un catalyseur et d'un solvant, caractérisé en ce que le catalyseur est le sulfate d'ammonium ou un sulfate double d'ammonium et d'un métal de transition.

2. Procédé selon la revendication 1 caractérisé en ce que le métal de transition est choisi parmi le chrome, le rhodium, le vanadium et le fer.

3. Procédé selon l'une des revendications 1 ou 2, caractérisé en ce que le sulfate double d'ammonium et d'un métal de transition est de type alun.

4. Procédé selon l'une quelconque des revendications 1 à 3, caractérisé en ce que le rapport molaire catalyseur/oléfine (ou alcool ou oléfine + alcool) est compris entre 0,01 et 0,5.

5. Procédé selon l'une quelconque des revendications 1 à 4, caractérisé en ce que le rapport molaire $NH_3$ (ou amine) / oléfine (ou alcool ou oléfine + alcool) est compris entre 1 et 10.

6. Procédé selon la revendication 5, caractérisé en ce que le rapport molaire $NH_3$ (ou amine) / oléfine (ou alcool ou oléfine + alcool) est supérieur à 2.

7. Procédé selon l'une quelconque des revendications 1 à 6, caractérisé en ce que la réaction de l'oléfine, de l'alcool ou de l'oléfine et de l'alcool en mélange, avec l'ammoniac ou l'amine primaire ou secondaire, est effectuée à une température comprise entre 200°C et 450°C sous pression autogène.

8. Procédé selon l'une quelconque des revendications 1 à 7, caractérisé en ce que le solvant est l'eau ou l'alcool correspondant à l'hydratation de l'oléfine, ou leurs mélanges.

9. Procédé selon l'une quelconque des revendications 1 à 8, caractérisé en ce que l'oléfine est l'isobutène et l'alcool le tertiobutanol.

## Patentansprüche

1. Verfahren zur Herstellung von Aminen durch Reaktion eines Monoolefins oder des daraus durch Hydratation erhältlichen entsprechenden Alkohols oder eines Gemischs eines Monoolefins und dieses Alkohols bei erhöhter Temperatur mit Ammoniak oder einem primären oder sekundären Amin in Gegenwart eines Katalysators und eines Lösungsmittels, dadurch gekennzeichnet, daß der Katalysator Ammoniumsulfat oder ein Doppelsulfat von Ammonium und einem Übergangsmetall ist.

2. Verfahren nach Anspruch 1, dadurch gekennzeichnet, daß das Übergangsmetall ausgewählt wird aus Chrom, Rhodium, Vanadium und Eisen.

3. Verfahren nach einem der Ansprüche 1 oder 2, dadurch gekennzeichnet, daß das Doppelsulfat von Ammonium und einem Übergangsmetall ein solches vom Alauntyp ist.

4. Verfahren nach einem der Ansprüche 1 bis 3, dadurch gekennzeichnet, daß das Molverhältnis Katalysator/Olefin (oder Alkohol oder Olefin + Alkohol) zwischen 0,01 und 0,5 beträgt.

5. Verfahren nach einem der Ansprüche 1 bis 4, dadurch gekennzeichnet, daß das Molverhältnis $NH_3$ (oder Amin) / Olefin (oder Alkohol oder Olefin + Alkohol) zwischen 1 und 10 liegt.

6. Verfahren nach Anspruch 5, dadurch gekennzeichnet, daß das Molverhältnis $NH_3$ (oder Amin) / Olefin (oder Alkohol oder Olefin + Alkohol) größer ist als 2.

7. Verfahren nach einem der Ansprüche 1 bis 6, dadurch gekennzeichnet, daß die Reaktion des Olefins, des Alkohols oder des Olefin- Alkoholgemisches mit Ammoniak oder dem primären oder sekundären Amin bei einer Temperatur zwischen 200°C und 450°C unter sich selbst einstellenden Druck durchgeführt wird.

8. Verfahren nach einem der Ansprüche 1 bis 7, dadurch gekennzeichnet, daß das Lösungsmittel Wasser oder der entsprechende, bei der Hydratation des Olefins erhältliche Alkohol oder deren Gemisch ist.

9. Verfahren nach einem der Ansprüche 1 bis 8, dadurch gekennzeichnet, daß das Olefin Isobuten und der Alkohol tert.-Butanol ist.

## Claims

1. Process for the manufacture of amines by a high-temperature reaction of a monoolefin or of the alcohol corresponding to its hydration or of a mixture of a monoolefin and of the said alcohol, with ammonia or a primary or secondary amine, in the presence of a catalyst and of a solvent, characterised in that the catalyst is ammonium sulphate or an ammonium transition metal double sulphate.

2. Process according to Claim 1, characterised in that the transition metal is chosen from chromium, rhodium, vanadium and iron.

3. Process according to either of Claims 1 and 2, characterised in that the ammonium transition metal double sulphate is of the alum type.

4. Process according to any one of Claims 1 to 3, characterised in that the molar ratio catalyst/olefin (or alcohol or olefin plus alcohol) is between 0.01 and 0.5.

5. Process according to any one of Claims 1 to 4, characterised in that the molar ratio $NH_3$ (or amine) / olefin (or alcohol or olefin plus alcohol) is between 1 and 10.

6. Process according to Claim 5, characterised in that the molar ratio $NH_3$ (or amine) / olefin (or alcohol or olefin plus alcohol) is higher than 2.

7. Process according to any one of Claims 1 to 6, characterised in that the reaction of the olefin, of the

alcohol or of the olefin and of the alcohol as a mixture, with the ammonia or the primary or secondary amine is carried out at a temperature of between 200°C and 450°C at autogenous pressure.

8. Process according to any one of Claims 1 to 7, characterised in that the solvent is water or the alcohol corresponding to the hydration of the olefin, or mixtures thereof.

9. Process according to any one of Claims 1 to 8, characterised in that the olefin is isobutene and the alcohol is tert-butanol.